# EUROPÄISCHE PATENTSCHRIFT

(11) **EP 2 405 842 B1**
(45) Veröffentlichungstag und Bekanntmachung des Hinweises auf die Patenterteilung: **04.09.2019**
(21) Anmeldenummer: 10710781.5
(22) Anmeldetag: 25.02.2010
(51) Int. Cl.: A61B 18/12

(54) **HOCHFREQUENZCHIRURGIEGENERATOR MIT EINEM ZUSATZTRANSFORMATOR**
HIGH FREQUENCY ELECTROSURGICAL GENERATOR WITH AN ADDITIONAL TRANSFORMER
GÉNÉRATEUR ÉLECTROCHIRURGICAL HAUTE FRÉQUENCE AVEC UN TRANSFORMATEUR SUPPLÉMENTAIRE

(30) Priorität: 11.03.2009 DE 102009012600
(43) Veröffentlichungstag der Anmeldung: 18.01.2012
(73) Patentinhaber: Erbe Elektromedizin GmbH, 72072 Tübingen (DE)
(72) Erfinder: SCHALL, Heiko, 72622 Nürtingen (DE); WERNER, Erich, 72827 Wannweil (DE); KEGREISS, Marc, 72072 Tübingen (DE); BELLER, Jürgen, 72810 Gomaringen (DE)
(74) Vertreter: Bohnenberger, Johannes
(86) Internationale Anmeldenummer: PCT/EP2010/001178
(87) Internationale Veröffentlichungsnummer: WO 2010/102725

(56) Entgegenhaltungen:
- DE-A1- 2 526 928
- US-A- 2 002 119
- US-A1- 2002 118 000
- US-A1- 2009 036 939
- US-B1- 6 508 815

## Beschreibung

Die Erfindung betrifft ein HF-Chirurgiegerät nach dem Oberbegriff des Patentanspruches 1. Ein derartiges HF-Chirurgiegerät ist beispielsweise aus DE 25 26 928 A1 bekannt.

HF-Chirurgiegeräte sind für eine Vielzahl von Einsatzmöglichkeiten bekannt. Bei einigen dieser Einsatzmöglichkeiten liegt die Lastimpedanz zwischen 0,1 und 1 kΩ, bei anderen Einsatzmöglichkeiten kann die Lastimpedanz auch niedriger werden. Insbesondere dann, wenn mit einem bipolaren Instrument eine Prostataresektion (TUR) unter gut leitfähiger Spülflüssigkeit (NaCl 0,9 %) durchgeführt wird, beträgt die Lastimpedanz nur noch etwa 20 Ω.

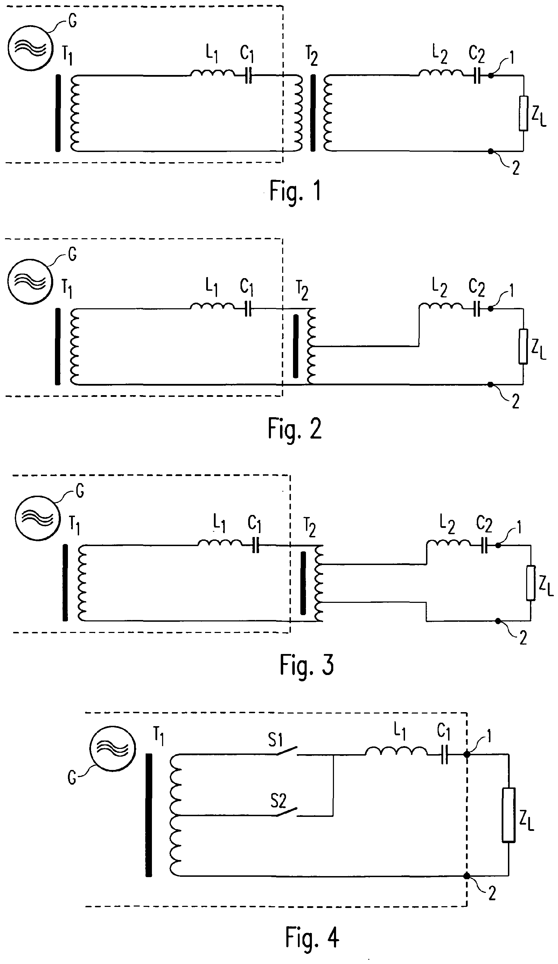

Um bei einem herkömmlichen HF-Chirurgiegerät, wie dies in Fig. 4 gezeigt ist, eine Lastanpassung vornehmen zu können, ist in dem HF-Chirurgiegerät (das in Fig. 4 mit einer unterbrochenen Linie angedeutet ist) ein Generator G vorgesehen, der einen Ausgangstransformator T₁ aufweist, welcher über eine Induktivität L₁ und eine Kapazität C₁, die zusammen einen Serienschwingkreis bilden, Ausgangsklemmen 1, 2 speist, an denen der Lastwiderstand ZL hängt. Bei einer hochohmigen Last wird ein erster Schalter S1 geschlossen, so dass die gesamte Sekundärwicklung des Transformators T₁ über den Serienschwingkreis mit dem Lastwiderstand ZL gekoppelt ist. Ist die Lastimpedanz ZL niedrig, so wird nur ein Teil der Sekundärwicklung des Transformators T₁ über den Schalter S2 an die Last ZL gekoppelt. Für diesen Fall ist zwar eine Verbesserung des Anpassungsverhältnisses der Impedanzen gewährleistet, jedoch muss der Transformator T₁ mit einer Wicklung versehen werden, die für den Abgriff des Schalters S2 einen größeren Drahtquerschnitt hat als für den Rest der Windungen oder sogar insgesamt einen größeren Drahtquerschnitt. Dies ist natürlich nachteilig. Weiterhin ist die Anpassung des Serienschwingkreises, die ja nicht nur in Abhängigkeit von der Generatorfrequenz, sondern auch in Abhängigkeit von der Last ZL zu erfolgen hat, in der einen oder der anderen Schalterstellung nicht mehr gegeben.

Die eingangs genannte DE 25 26 928 A1 betrifft ein bipolares HF-chirurgisches Instrument für die Koagulation. Dort wird als nachteilig beschrieben, dass der elektrische Widerstand des Gewebes während der Koagulation zunimmt. Hierbei kann es zu einem Funkenüberschlag über diejenigen Elektroden kommen, die einen höheren Übergangswiderstand zu dem Gewebe haben. Eine gleichmäßige Koagulation wird dadurch erheblich erschwert.

Die in DE 25 26 928 A1 beschriebene Vorrichtung soll einen Funkenüberschlag zwischen der Elektrode und dem Gewebe verhindern, wenn der Gewebewiderstand während der Behandlung ansteigt. Bei dem bekannten Gerät wird dazu eine Ausgangsspannung konstant gehalten, um einen zu großen Spannungsabfall zwischen den Elektroden und damit einen Funkenüberschlag zu vermeiden, wenn der Gewebewiderstand zunimmt.

Der Erfindung liegt die Aufgabe zu Grunde, ein HF-Chirurgiegerät der eingangs genannten Art dahingehend aufzuzeigen, dass eine Anpassung auch an niedrige Lasten ermöglicht wird.

Diese Aufgabe wird durch ein HF-Chirurgiegerät nach Anspruch 1 gelöst.

Insbesondere wird die genannte Aufgabe durch ein HF-Chirurgiegerät gelöst, umfassend einen HF-Generator mit einer Ausgangsschaltung, die einen Ausgangstransformator enthält und eine Ausgangsimpedanz aufweist, und mit Ausgangsklemmen, an denen ein HF-chirurgisches Instrument angeschlossen werden kann, das einen Hochfrequenzstrom mit einer bestimmten Frequenz zur Behandlung eines Gewebes in dieses liefert, wobei eine Zusatzausgangsschaltung vorgesehen ist, die einen Zusatztransformator enthält, der zwischen die Ausgangsschaltung und die Ausgangsklemmen unter Verringerung der Ausgangsimpedanz geschaltet ist.

Diese Zusatzausgangsschaltung kann sowohl als externes Zusatzgerät ausgeführt als auch im HF-Chirurgiegerät angebracht sein. Dadurch wird in einfacher Weise gewährleistet, dass ein herkömmliches Chirurgiegerät für besondere Zwecke (bei niederohmiger Last) verwendet werden kann. Der erforderliche Aufwand ist gering.

Vorzugsweise ist der Zusatztransformator als Spartransformator ausgeführt, so dass die Potentialtrennung durch im HF-Chirurgiegerät vorgesehenen Transformator erfolgt. Dieser Zusatztransformator kann eine asymmetrische oder aber, bevorzugterweise, eine symmetrische Wicklung aufweisen.

Der Zusatztransformator weist vorzugsweise einen geringen, insbesondere aber keinen Luftspalt auf. Dadurch kann die Anpassung optimiert werden. Weiterhin ist der Zusatztransformator vorzugsweise streuarm ausgebildet.

Die Induktivität des Zusatztransformators liegt vorzugsweise über 1 mH.

Die Zusatzschaltung weist weiterhin einen Kondensator zwischen dem Zusatztransformator und den Ausgangsklemmen auf. Erfindungsgemäß ist hier jedoch ebenfalls ein Serienschwingkreis vorgesehen, und zwar derart, dass dessen Resonanzfrequenz über der bestimmten Frequenz des Generators liegt. Dadurch wird die Anpassung an die Last weiterhin verbessert.

Nachfolgend wird ein Ausführungsbeispiel der Erfindung anhand von Abbildungen näher erläutert. Hierbei zeigen
- - Fig. 1: ein Schaltbild einer ersten Ausführungsform der Erfindung,
- - Fig. 2: ein Schaltbild einer zweiten Ausführungsform der Erfindung,
- - Fig. 3: ein Schaltbild einer dritten Ausführungsform der Erfindung und
- - Fig. 4: ein Schaltbild einer Anordnung, welche nur mit dem Ausgangstransformator des HF-Chirurgiegerätes arbeitet.

In der nachfolgenden Beschreibung werden für gleiche und gleich wirkende Teile dieselben Bezugsziffern verwendet.

Aus Fig. 1 geht hervor, dass das HF-Chirurgiegerät einen Generator G umfasst, der einen Ausgangstransformator T₁ und einen Serienschwingkreis L1, C1 als Ausgangsschaltung aufweist. Diese Anordnung ist an einen Zusatztransformator T2 gekoppelt, der primär eine höhere Windungszahl als sekundär hat. Der Zusatztransformator T2 ist über einen weiteren Serienschwingkreis L2, C2 mit Ausgangsklemmen 1, 2 verbunden, an denen eine Last ZL anliegt. Durch den Zusatztransformator T2 wird der Innenwiderstand der Gesamtanordnung herabgesetzt und zwar derart, dass im Wesentlichen eine Anpassung an die Lastimpedanz ZL erfolgt. Der Zusatzserienschwingkreis L2, C2 ist so ausgelegt, dass unter Einbeziehung der Ausgangsfrequenz des Generators G und der Last ZL eine Anpassung vorliegt.

Die in Fig. 2 gezeigte Ausführungsform unterscheidet sich von der nach Fig. 1 dadurch, dass der Zusatztransformator T2 als Spartransformator ausgebildet ist. Die Ausführungsform nach Fig. 3 wiederum unterscheidet sich von der nach Fig. 2 dadurch, dass dieser Spartransformator T2 einen symmetrischen Abgriff zur Ankopplung der Last ZL (über den Serienschwingkreis L2, C2) aufweist.

## Patentansprüche

1. HF-Chirurgiegerät, umfassend
- einen HF-Generator (G) mit
o einer Ausgangsschaltung, die einen Ausgangstransformator (T₁) enthält und eine Ausgangsimpedanz aufweist, und mit
o Ausgangsklemmen (1, 2), an denen ein HF-chirurgisches Instrument angeschlossen werden kann, das einen Hochfrequenzstrom mit einer bestimmten Frequenz zur Behandlung eines Gewebes (ZL) liefert,
- eine Zusatzausgangsschaltung, die einen Zusatztransformator (T2) enthält, der zwischen die Ausgangsschaltung (T1, L1, C1) und die Ausgangsklemmen (1, 2) unter Verringerung der Ausgangsimpedanz gegenüber der Ausgangsimpedanz der Ausgangsschaltung geschaltet ist, sodass eine Anpassung des Innenwiderstands des HF-Chirurgiegeräts an die Lastimpedanz (ZL) erfolgt,
wobei die Primärseite des Zusatztransformators (T2) eine höhere Windungszahl aufweist als seine Sekundärseite,
**dadurch gekennzeichnet, dass**
die Zusatzausgangsschaltung einen Serienschwingkreis (L2, C2) umfasst derart, dass dessen Resonanzfrequenz über der bestimmten Frequenz des Generators liegt.

2. HF-Chirurgiegerät nach Anspruch 1,
**dadurch gekennzeichnet, dass**
der Zusatztransformator (T₂) als Spartransformator ausgeführt ist.

3. HF-Chirurgiegerät nach Anspruch 2,
**dadurch gekennzeichnet, dass**
der Zusatztransformator (T₂) symmetrische Wicklungsverhältnisse aufweist.

4. HF-Chirurgiegerät nach einem der vorhergehenden Ansprüche,
**dadurch gekennzeichnet, dass**
der Zusatztransformator (T₂) keinen Luftspalt aufweist.

5. HF-Chirurgiegerät nach einem der vorhergehenden Ansprüche,
**dadurch gekennzeichnet, dass**
der Zusatztransformator (T₂) streuarm ausgebildet ist.

6. HF-Chirurgiegerät nach einem der vorhergehenden Ansprüche,
**dadurch gekennzeichnet, dass**
der Zusatztransformator (T₂) eine Induktivität von über 1 mH aufweist.

7. HF-Chirurgiegerät nach einem der vorhergehenden Ansprüche,
**dadurch gekennzeichnet, dass**
die Zusatzschaltung einen Kondensator (C₂) zwischen dem Zusatztransformator (T₂) und den Ausgangsklemmen (1, 2) umfasst.

## Claims

1. Electrosurgical device, comprising
an RF generator (G) with an output circuit containing an output transformer (T₁) and having an output impedance, and with output terminals (1, 2), to which an electrosurgical instrument can be connected, the latter supplying a radiofrequency current at a certain frequency for treating a tissue (Z_{L}),
an additional output circuit containing an additional transformer (T₂), which is connected between the output circuit (T₁, L₁, C₁) and the output terminals (1, 2) with reduction of the output impedance in relation to the output impedance of the output circuit such that the output resistance of the electrosurgical device is adapted to the load impedance (Z_{L}), wherein the primary side of the additional transformer (T₂) has a greater number of turns per unit length than a secondary side and **characterized in that**
the additional output circuit comprises a resonant circuit (L₂, C₂) such that the resonant frequency thereof lies above the certain frequency of the generator.

2. Electrosurgical device according to Claim 1, **characterized in that**
the additional transformer (T₂) is embodied as an autotransformer.

3. Electrosurgical device according to Claim 2, **characterized in that**
the additional transformer (T₂) has symmetric winding ratios.

4. Electrosurgical device according to any one of the preceding claims,
**characterized in that**
the additional transformer (T₂) has no air gap.

5. Electrosurgical device according to any one of the preceding claims,
**characterized in that**
the additional transformer (T₂) has a low-leakage embodiment.

6. Electrosurgical device according to any one of the preceding claims,
**characterized in that**
the additional transformer (T2) has an inductance of more than 1 mH.

7. Electrosurgical device according to any one of the preceding claims,
**characterized in that**
the additional circuit comprises a capacitor (C₂) between the additional transformer (T₂) and the output terminals (1, 2).

## Revendications

1. Appareil chirurgical haute fréquence, comprenant
un générateur haute fréquence (G) avec un circuit de sortie, qui comprend un transformateur de sortie (T₁) et qui présente une impédance de sortie, et avec des bornes de sortie (1, 2), auxquelles peut être connecté un instrument chirurgical haute fréquence, qui fournit un courant haute fréquence avec une fréquence déterminée pour le traitement d'un tissu (Z_{L}),
un circuit de sortie supplémentaire, qui contient un transformateur supplémentaire (T₂), qui est monté entre le circuit de sortie (T₁, L₁, C₁) et les bornes de sortie (1, 2) avec réduction de l'impédance de sortie par rapport à l'impédance de sortie du circuit de sortie, de telle manière qu'il se produise une adaptation de la résistance interne de l'appareil chirurgical haute fréquence à l'impédance de charge (Z_{L}), dans lequel le côté primaire du transformateur supplémentaire (T₂) présente un nombre de spires plus élevé que son côté secondaire, et
**caractérisé en ce que** le circuit de sortie supplémentaire comprend un circuit oscillant en série (L₂, C₂), de telle manière que sa fréquence de résonance se situe en dessous de la fréquence déterminée du générateur.

2. Appareil chirurgical haute fréquence selon la revendication 1, **caractérisé en ce que** le transformateur supplémentaire (T₂) est formé par un autotransformateur.

3. Appareil chirurgical haute fréquence selon la revendication 2, **caractérisé en ce que** le transformateur supplémentaire (T₂) présente des rapports d'enroulement symétriques.

4. Appareil chirurgical haute fréquence selon l'une quelconque des revendications précédentes, **caractérisé en ce que** le transformateur supplémentaire (T₂) ne présente pas d'entrefer.

5. Appareil chirurgical haute fréquence selon l'une quelconque des revendications précédentes, **caractérisé en ce que** le transformateur supplémentaire (T₂) est réalisé à faible dispersion.

6. Appareil chirurgical haute fréquence selon l'une quelconque des revendications précédentes, **caractérisé en ce que** le transformateur supplémentaire (T₂) présente une inductance de plus de 1 mH.

7. Appareil chirurgical haute fréquence selon l'une quelconque des revendications précédentes, **caractérisé en ce que** le circuit supplémentaire comprend un condensateur (C₂) entre le transformateur supplémentaire (T₂) et les bornes de sortie (1, 2).
